(19) Europäisches Patentamt / European Patent Office / Office européen des brevets

(11) **EP 4 721 751 A1**

(12) **EUROPEAN PATENT APPLICATION**

(43) Date of publication:
08.04.2026 Bulletin 2026/15

(21) Application number: 25206946.3

(22) Date of filing: 06.10.2025

(51) International Patent Classification (IPC):
*A61K 38/00* (2006.01)   *A61K 8/64* (2006.01)
*A61P 17/02* (2006.01)   *A61P 17/16* (2006.01)
*C07K 14/00* (2006.01)

(52) Cooperative Patent Classification (CPC):
(C-Sets available)
**A61K 38/00; A61K 8/64; A61P 17/02; A61P 17/16**
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 07.10.2024  PL 44996424

(71) Applicant: **Gdanski Uniwersytet Medyczny**
**80-210 Gdansk (PL)**

(72) Inventors:
 • **PIKULA, Michal**
  **80-170 Gdansk (PL)**
 • **DEPTULA, Milena**
  **80-174 Gdansk (PL)**

 • **SKONIECKA, Aneta**
  **80-180 Gdansk (PL)**
 • **ZAWRZYKRAJ, Malgorzata**
  **83-034 Trabki Wielkie (PL)**
 • **ZIELINSKI, Jacek**
  **83-110 Tczew (PL)**
 • **DIERZYNSKA, Maria**
  **81-824 Sopot (PL)**
 • **SAWICKA, Justyna**
  **80-174 Gdansk (PL)**
 • **RODZIEWICZ-MOTOWIDLO, Sylwia**
  **83-010 Rotmanka (PL)**

(74) Representative: **Godlewski, Piotr**
**FGGH IP**
**Kancelaria Patentowa Piotr Godlewski**
**Ul. Bociania 19/8**
**02-807 Warszawa (PL)**

(54) **PHARMACEUTICAL COMPOSITION WITH PROTECTIVE AND PRO-REGENERATIVE EFFECTS AND ITS USE**

(57)   The invention provides a pharmaceutical composition comprising IM2 peptide of SEQ ID NO: 1 and NE1 peptide of SEQ ID NO: 2, and at least one pharmaceutically acceptable excipient, and its use.

Fig. 1

(52) Cooperative Patent Classification (CPC): (Cont.)

C-Sets
**A61K 38/00, A61K 2300/00**

**Description**

The invention concerns a pharmaceutical composition exhibiting protective and pro-regenerative properties, as well as its use. The invention relates to the field of medicine, particularly dermatology, and is intended for use in skin care and protection, especially following radiotherapy and chemotherapy.

Polish patent PL237242 B1 describes the RDKVYR peptide or a pharmaceutically acceptable salt thereof for use as a means of stimulating complex tissue regeneration or healing wounds caused by mechanical and/or chemical and/or thermal and/or radiation damage and/or surgical procedures and/or other pathological conditions.

Polish patent PL237001 B1 describes a peptide compound with the general formula $R-(G)_3-B-(G)_3-O-NH_2$, where: R denotes the amino acid sequence RADARADARADARADA, B denotes the AAPV amino acid sequence specific for neutrophil elastase, G denotes the amino acid glycine, and O denotes an amino acid sequence biologically active in accelerating skin wound healing. It is also known for its use in promoting wound healing and wound regeneration, including for the regeneration of skin and muscle tissue, particularly for promoting the healing of wounds caused by mechanical, chemical, thermal, or radiation damage, surgical procedures, or pathological conditions.

Polish patent PL241125 B1 describes a pharmaceutical composition that contains zebularine and retinoic acid or pharmaceutically acceptable salts thereof and at least one pharmaceutically acceptable carrier or diluent for use as an agent for stimulating regeneration or healing of wounds caused by mechanical, chemical, thermal damage, surgical operations or pathological conditions.

Currently, the pharmaceutical market, especially the dermatological market, offers very few cosmetics or medical products specifically dedicated to the care and protection of the skin of oncological patients (e.g., Radiocalm, Bioderma Cicabio Restor, Pharmaceris X-Rays Liposubtilium, Radioprotect). Oncological radiotherapy and chemotherapy have a wide range of negative effects on human skin. Additionally, the condition of the skin after radiotherapy is a problem resulting from the technical limitations of the therapy used; its tenderness and sensitivity pose a challenge during application [L. Lorenzo-Gallego et al., Changes in Pain Sensitivity in Treatment for Breast Cancer: A 12-Month Follow-Up Case Series, Int. J. Environ. Res. Public Health 2022, 19(7), 4055]. The skin of oncology patients requires special treatment that ensures protection, hydration, and the possibility of tissue reconstruction while facilitating application [S. A. Baskin et al., The neoadjuvant approach to treatment of breast cancer: Multidisciplinary management to improve outcomes, Surgical Oncology Insight 2024, 1, 100059].

In chronic wounds, the wound healing process is impaired, which leads to long-term, difficulties in treatment. Standard wound therapy methods, including the use of wound dressings or negative pressure therapy, improve the wound environment but do not stimulate more effective healing [M.D. Yue-Yung Hu et al., Impact of neoadjuvant chemotherapy on breast reconstruction, Cancer 2011, 117(13), 2827-3060]. Therefore, alternative products are sought that also contain ingredients that stimulate the regenerative process. Furthermore, oncological treatments, primarily radiotherapy and chemotherapy, as well as surgical procedures, are associated with a high risk of skin lesions and defects [H. Matsuki et al., Pitfall in the Surgical Management of a Shrunken Skin Defect after Neoadjuvant Chemotherapy for Locally Advanced Breast Cancer, Case Rep Oncol 2022, 15 (3): 1101-1106].

The aim of this invention was to develop a pharmaceutical composition containing dermatological compounds that, when applied topically to skin damaged by neoadjuvant therapy, would both protect and promote skin regeneration. Another aim of the invention was to develop a product and application method for the pharmaceutical composition that is easy to use and allows for painless, even, and controlled application on the affected skin.

All of the above technical challenges have been resolved with this invention.

The invention provides a pharmaceutical composition comprising the IM2 peptide of SEQ ID NO:1 and the NE1 peptide of SEQ ID NO:2, and at least one pharmaceutically acceptable excipient.

Preferably, in the composition of the invention, the concentration of the NE1 peptide is from 0.1 to 25 µg/ml, and the concentration of IM2 is from 0.1 to 150 µg/ml.

Preferably, the composition of the invention contains poloxamer P-407 in an amount of 1% by weight, based on the total weight of the composition.

Preferably, the pharmaceutical composition of the invention contains a preservative in an amount of 1% to 1.5% by weight, based on the total weight of the composition.

Preferably, the preservative is a mixture of potassium sorbate and sodium benzoate.

Preferably, it contains potassium sorbate in an amount of 14.3 to 15.7% by weight and sodium benzoate in an amount of 28.5 to 31.5% by weight.

Preferably, the composition of the invention is in the form of an emulsion, aerosol, or aqueous solution.

Another subject of the invention is use of the pharmaceutical composition as a preparation for external use in the care and protection of skin after radiotherapy, burns, and other irritants such as sunlight or temperature.

Preferably, the composition of the invention is used in the form of an aerosol.

According to the present invention, the peptide named IM2 refers to an analogue of the immunofan (IM) peptide, while the peptide named NE1 refers to an analogue of the GHK peptide with a duplicated amino acid sequence.

The pharmaceutical composition according to the invention is characterized by biocompatibility, bioactivity, and biodegradability. The advantage of the composition according to the invention is its liquid form, which allows for atomization, enabling painless and even application to sensitive skin after radiotherapy, burns, and exposure to other irritants (sunlight, temperature).

The composition according to the invention is a potential cosmetic for use on sensitive skin, with particular emphasis on oncological patients. Studies conducted on probands (volunteers) demonstrated soothing and moisturizing properties, and the aerosol form provides ease of application, particularly on the delicate skin of individuals after chemotherapy/radiotherapy. Studies were conducted on volunteers (external service), most of whom had a history of allergies. A soothing effect, reduced redness, and reduced skin tightness were observed in most volunteers. In vitro laboratory studies, conducted, among others, on skin cell lines (keratinocytes and fibroblasts) showed no negative effects of the active compounds used in the formulation. At the same time, analysis of the complex's effect on cell migration demonstrated a stimulating effect on this process, which is an important aspect in skin regeneration. The safety of the composition was further confirmed by studies of allergenic potential (in vitro tests), thus the composition according to the invention has positive effects at both the cellular and clinical levels.

The composition according to the invention can be used in hospital settings as well as by private consumers. Administration of the cosmetic in aerosol form allows for effective, more hygienic, and less painful use by the patient.

The invention is presented in the following figures:

Fig. 1     presents A) the mass spectrum of the NE1 peptide; B) the mass spectrum of the IM2 peptide;

Fig. 2     presents the evaluation of the effect of the IM2 and NE1 peptides, their mixture, P407, and the test product on the proliferation of skin cells (46BR.1N fibroblast cell line, HaCaT keratinocytes, and primary fibroblasts) after 24 hours of incubation. * - statistically significant differences, Mann-Whitney U test, $p < 0.05$;

Fig. 3     presents the results of the assessment of allergenic potential using the basophil activation test (BAT);

Fig. 4     presents the release profile of the NE1 and IM2 peptides from the pharmaceutical composition;

Fig. 5     shows the results of assessing the effect of peptides IM2+NE1, P407, and the test product on HaCaT keratinocyte migration after 24 hours of incubation. * - statistically significant differences, Mann-Whitney U test, $p < 0.05$, FBS - positive control, cells incubated in standard medium with 10% FBS;

Fig. 6     presents the results of application studies with the pharmaceutical composition of the invention;

Fig. 7     presents the levels of secreted cytokines IL-6 (A), IL-8 (B), VEGF-A (C), and TNF-$\alpha$ (D) in HaCaT cells after stimulation with the peptide mixture, poloxamer P407, and the pharmaceutical composition of the invention;

Fig. 8     presents the level of cytokines IL-6 (A), IL-8 (B), FGF-2 (C), and VEGF-A (D) in 46BR.1N fibroblast cells after stimulation with a mixture of peptides, poloxamer P407 and a pharmaceutical composition according to the invention.

The invention is presented in the following examples of embodiments, which, however, are not of a limiting nature and are intended only to illustrate the invention.

**Example 1**

Peptide Synthesis

The synthesis of peptide IM2 with the sequence $NH_2$-RDKVAR-OH (SEQ ID NO:1) and peptide NE1 with the sequence Ac-GHKGGGAAPVGGGHK-$NH_2$ (SEQ ID NO:2) was performed on a solid support according to Fmoc chemistry methodology using a P11 synthesizer (Activotec). The amide resin R RAM (TentaGel®) was used for the synthesis, with a deposition degree of 0.18 mmol/g for the NE1 peptide, and Fmoc-D-Arg(Pbf)-TentaGel S AC for the IM2 peptide.

Reagents used for synthesis:

- DMF
- 0.2 M solutions of $N^{\alpha}$-Fmoc-derivative amino acids in DMF:
- Fmoc-Gly-OH;

- Fmoc-His(TrT)-OH;
- Fmoc-Lys(Boc)-OH;
- Fmoc-Ala-OH
- Fmoc-Pro-OH;
- Fmoc-Val-OH;
- 20% piperidine solution in DMF;
- 0.5 M Oxyma pure in DMF;
- 0.5 M N-acetylimidazole in DMF;
- 0.5 M DIC in DMF.

Peptide Cleavage from the Support

A mixture of 88% TFA (Sigma-Aldrich) + 5% $H_2O$ + 5% phenol + 2% TIPSI (Sigma-Aldrich) (v/v/v/v) was used to cleave the peptide from the support while simultaneously removing the side shields. The reaction was carried out for three hours using a laboratory shaker, after which the resin was filtered under reduced pressure, and the filtrate was evaporated to dryness in a vacuum evaporator. The resulting precipitate was covered with cold diethyl ether, triturated, and then centrifuged (20 min). The precipitate was washed three times with $Et_2O$ to remove impurities (centrifuging after each wash) and then allowed to dry in a vacuum desiccator. The resulting crude product (peptide) was dissolved in water and lyophilized.

Characterization of the Obtained Product - HPLC and LC-MS

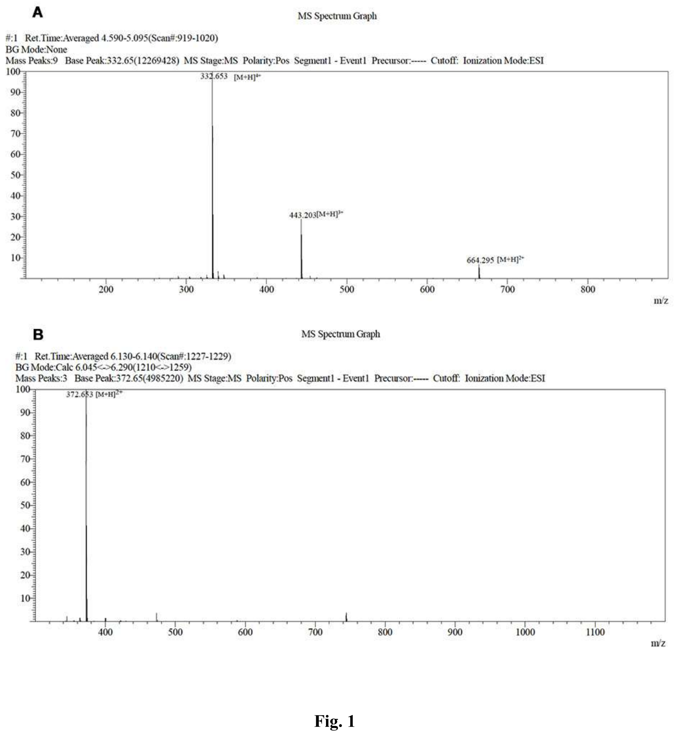

The obtained crude product (peptide) was analyzed by chromatography using reversed-phase high-performance liquid chromatography (RP-HPLC). Column: Luna $5\mu$m C18, 100 Å, 250 x 4.6 mm. Phase system: A: 0.1% TFA in water, B: 0.1% TFA in 80% acetonitrile in water, flow rate: 1 ml/min, UV detection at 223 nm. LC-MS ESI TOF analysis using a Kromasil C8 column, $5\mu$m 100 Å (250 mm x 1 mm). Figure 1 presents the MS spectra of both peptides, where in Figure 1A (peptide NE1) multiply charged ions are visible with the following mass to charge ratio (m/z): 332.653 $[M+H]^{4+}$ 443.203 $[M+H]^{3+}$ 664.295 $[M+H]^{2+}$. In the spectrum of Figure 1B, a multiply charged ion is visible with the mass to charge ratio of 372.653 $[M+H]^{2+}$.

Trifluoroacetate Counterion Exchange for Acetate

The trifluoroacetate counterion exchange for acetate was performed on a Strata C18-E column (55 $\mu$m, 70 Å) according to the following procedure:

- The peptide dissolved in water was applied to the equilibrated column.

- The progress of peptide binding to the column bed was monitored using reversed-phase high-performance liquid chromatography.

- After the peptide bound to the column, it was washed with a 0.1 M aqueous ammonium acetate solution.

- The peptide was eluted with a 0.1 M ammonium acetate solution in MeOH.

The resulting eluate was evaporated using a vacuum evaporator, and then the excess ammonium acetate was sublimated on a lyophilizer. To remove residual ammonium, the lyophilisate was re-dissolved in water and lyophilized again.

**Example 2**

Method of preparing a composition according to the invention

To prepare the composition according to the invention, the following steps were performed. An appropriate amount of Poloxamer P-407 was weighed into a clean bottle, then the polymer was dissolved in miliQ water at 4°C. Next, the IM2 and NE1 peptides dissolved in miliQ water were added to the mixture. The solution was mixed until a homogeneous mixture was obtained. A food-grade preservative was added, and the entire solution was made up with an appropriate amount of miliQ water to form a mixture containing the appropriate percentage and weight concentrations (presented below).

Reagents used to prepare the composition:

- Peptide IM2 and NE1 in a 1:1 ratio, with the NE1 peptide concentration ranging from 0.1 to 25 $\mu$g/ml and the IM2 concentration ranging from 0.1 to 150 $\mu$g/ml.
- Poloxamer P-407 - 1.0%,
- Euxyl ® K712 food preservative in the form of a mixture of potassium sorbate (14.3-15.7% w/w) and sodium benzoate (28.5-31.5% w/w) - from 1.0% to 1.5%,
- Water

## Example 3

Safety Testing of the Composition of the Invention

*Analysis of the Effect of the Composition on Proliferation*

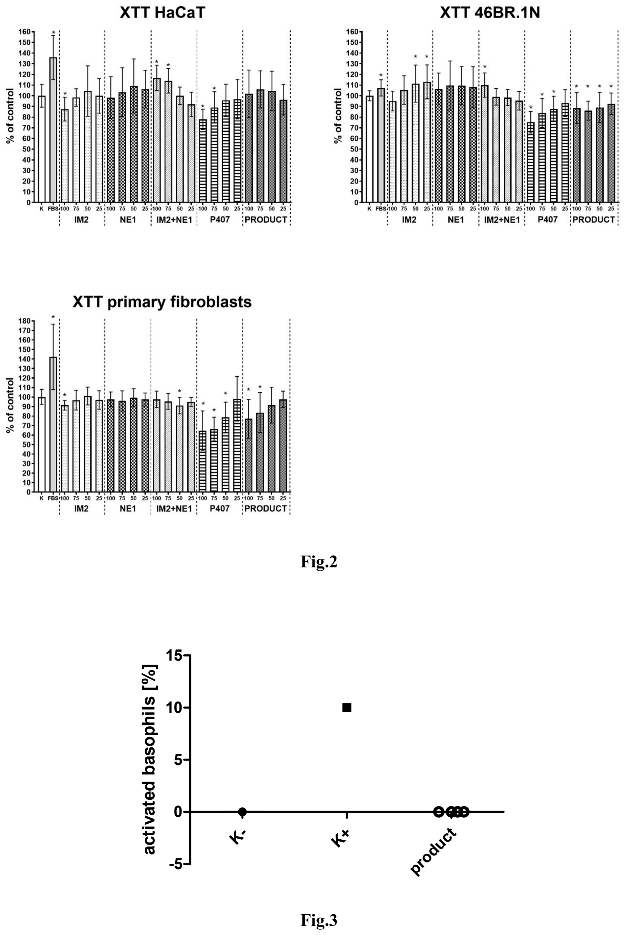

The effect of the prepared composition (peptides IM2 and NE1, both at a concentration of 10 $\mu$g/ml, 1% poloxamer P407, without preservatives already approved for use in cosmetics and food products and having no effect on the skin) on the proliferation of HaCaT keratinocyte lines, 46BR.1N fibroblasts, and primary dermal fibroblasts was assessed. The test was performed using an XTT kit. After 4 hours, spectrophotometric readings were taken at a wavelength of 450 nm. The experiment was performed in three replicates. Cell proliferation is shown in Fig. 2. According to the ISO 10933-5:2009 standard, a decrease in viability of more than 30% is considered cytotoxic. The results confirmed the safety of the peptide mixture and composition towards the tested cells (no decrease in proliferation greater than 30% was observed).

*In vitro basophil activation test (BAT)*

To test the allergenic potential of the new composition, an in vitro basophil activation test (BAT, DuraClone IF Basophil Activation Tube, Beckman Coulter C23406) was performed on whole blood from four healthy volunteers. Flow cytometric analysis demonstrated no in vitro basophil stimulation and, therefore, no allergenic potential for the test compound (while maintaining the correct results for the positive and negative controls). The obtained results are consistent with the observations made in the application study. The assessment of allergenic potential is presented in Fig. 3.

## Example 4

Study of the Physicochemical Properties and Microbiological Purity of the Composition of the Invention

*Analysis of Peptide Stability and Transdermal Diffusion*

Peptide Stability Studies in Water: Analysis of the stability of the IM2 and NE1 peptides was performed at the following time points: 1, 2, 3, 6, and 24 hours. The IM2 and NE1 peptides were incubated in miliQ water at 37°C with continuous shaking at 300 rpm. Samples collected at the appropriate time points were analyzed using high-performance liquid chromatography (HPLC) on a Phenomenex Luna C18(2) 4.6 x 250 mm, 5 $\mu$m, 100 Å column using a PDA detector. The systems consisted of 0.1% trifluoroacetic acid (TFA) in water (A) and 0.1% TFA in 80% ACN (B). Analysis was performed using a linear gradient of 5-100% B in 60 minutes at a flow rate of 1 mL/min. Calculations were performed using Shimadzu LCsolution software. Analysis of the obtained data confirmed that the NE1 and IM2 peptides were stable in water throughout the experiment.

*Transdermal Diffusion Studies*

The permeation studies of the peptides contained in the resulting composition, IM2 and NE1, were conducted using a Phoenix Dry Heat Systems system. The acceptor and donor chambers were separated by a Strat-M® membrane imitating human skin (Merck). 400 $\mu$l of the composition was applied to the donor chamber, while the acceptor chamber was filled with phosphate buffer (PBS) and heated to 37°C with constant stirring at 300 rpm. At 28 defined time points over a period of 420 minutes, 200 $\mu$l of the solution was withdrawn from the acceptor chamber and then topped up with 200 $\mu$l of fresh PBS solution.

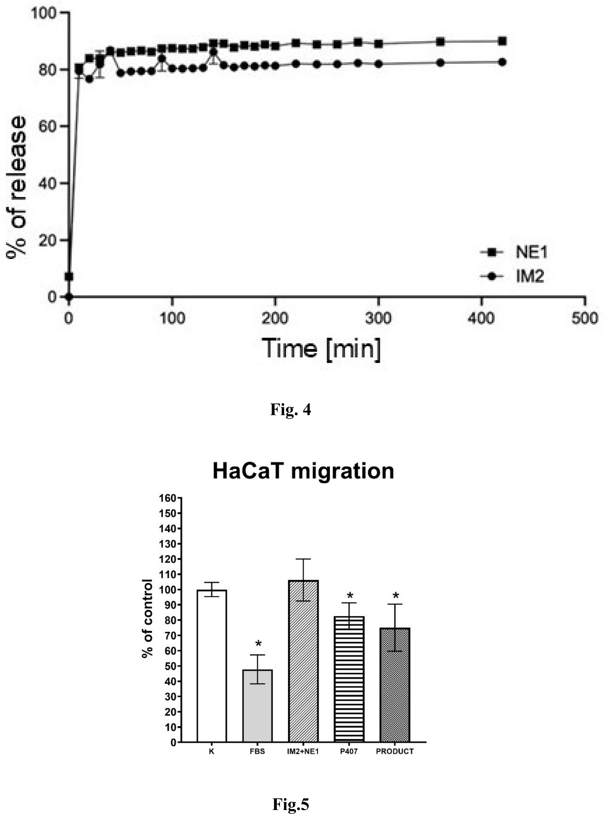

Figure 4 shows the release profile of the IM2 and NE1 peptides from the composition of the invention, where the data contain the mean value with standard error (SEM) from three replicates of the experiment. The NE1 peptide exhibits rapid membrane permeation, reaching 80% release from the formulation after 30 minutes and then stabilizing at a maximum release rate of 90%. Similarly, the IM2 peptide exhibited an immediate release rate of 80%, maintaining this rate throughout the experiment.

*Physicochemical Properties Testing*

Physicochemical testing (pH, viscosity, density) and stability and packaging compatibility testing were satisfactory. The results of the physicochemical properties assessment of the composition are as follows:

| Viscosity Density pH | 4.43±0.042 0.9888 5.5 | [mPas] [g/ml] |
| --- | --- | --- |

*Microbiological purity test with preservation test*

At the same time, external analyses necessary to introduce the composition to the market were carried out. The microbiological purity test together with the preservation test (challenge test) of the prepared composition met the required criteria (A and B) for the effectiveness of antimicrobial protection according to the PN-EN ISO 11930:2019 standard (Table 1-5)

Table 1. Evaluation of the neutralization effectiveness of the antimicrobial activity of the composition

| Validation of the neutralization of the preservative system in the test using Eugon LT100 | | | | |
| --- | --- | --- | --- | --- |
| Tested strains | Nvf (unit/ml*) | Nvn (unit/ml) | Nvf $\geq$ 0,5 Nvn* | Nv (unit/ml) |
| *S. aureus* ATCC 6538 | $3,5 \times 10^3$ | $5,2 \times 10^3$ | > 0,5 | $5,6 \times 10^3$ |
| *E. coli* ATCC 8739 | $3,3 \times 10^3$ | $4,9 \times 10^3$ | > 0,5 | $5,3 \times 10^3$ |
| *Ps. Aeruginosa* ATCC 9027 | $3,0 \times 10^3$ | $5,1 \times 10^3$ | > 0,5 | $5,4 \times 10^3$ |
| *C. albicans* ATCC 10231 | $3,3 \times 10^3$ | $5,3 \times 10^3$ | > 0,5 | $5,7 \times 10^3$ |
| *A. brasiliensis* ATCC 16404 | $2,5 \times 10^3$ | $3,4 \times 10^3$ | > 0,5 | $3,7 \times 10^3$ |
| *unit/ml - colony forming unit in 1ml ** Nvf $\geq$ 0.5 Nvn - assumption according to PN-EN ISO 11930:2019 | | | | |

Nvf - number of microorganisms in 1ml of a mixture consisting of: 1ml of the test sample + 9ml of neutralizer + 1ml of the test strain suspension (N) from a dilution of $10^{-4}$ (bacteria)/$10^{-3}$ (*C. albicans* and *A. brasiliensis*)

Nvn - Nvn - number of microorganisms in 1ml of a mixture of: 1ml of diluent + 9ml of neutralizer + of the test strain suspension (N) from a dilution of $10^{-4}$ (bacteria)/$10^{-3}$ (C. *albicans* and *A. brasiliensis*) Nv - number of microorganisms in the control: 10 ml of diluent + 1 ml of suspension of the test strain (N) from a dilution of $10^{-4}$ (bacteria)/$10^{-3}$ (C. *albicans and A. brasiliensis*)

Results:

The neutralization efficiency of the preservative system in the test sample was determined for the neutralizer: Eugon LT 100 at a sample dilution of $10^{-1}$. The assumption of **Nvf $\geq$ 0.5 Nvn** was met for all test strains used in the neutralization study. The neutralizer's effectiveness was assessed at above 50%.

Table 2. Challenge test

| Density of suspensions of test strains used for contamination of the tested samples | | |
| --- | --- | --- |
| Test strains | N (unit/ml) Number of microorganisms in a calibrated suspension | $N_0 = N/100$ (unit/ml) The number of microorganisms in the sample tested immediately after contamination ($T_0$) |
| *S. aureus* ATCC 6538 | $4,7 \times 10^7$ | $4,7 \times 10^5$ |
| *E. coli* ATCC 8739 | $4,2 \times 10^7$ | $4,2 \times 10^5$ |
| *Ps. Aeruginosa* ATCC 9027 | $4,0 \times 10^7$ | $4,0 \times 10^5$ |

(continued)

| Density of suspensions of test strains used for contamination of the tested samples | | |
|---|---|---|
| Test strains | N (unit/ml) Number of microorganisms in a calibrated suspension | $N_0$ = N/100 (unit/ml) The number of microorganisms in the sample tested immediately after contamination ($T_0$) |
| C. albicans ATCC 10231 | $3,6 \times 10^6$ | $3,6 \times 10^4$ |
| A. brasiliensis ATCC 16404 | $3,0 \times 10^6$ | $3,0 \times 10^4$ |

Results:

According to the PN-EN ISO 11930:2019 standard, nephelometric-calibrated suspensions of test microorganisms (N) with densities ranging from $1\times10^7$ to $1\times10^8$ cfu/ml for bacteria and $1\times10^6$ to $1\times10^7$ cfu/ml for fungi were used to contaminate the test samples. The results presented in Table 2 meet these assumptions.

Table 3. Number of microorganisms in 1 ml of sample at specified time intervals

| Test strains | $T_0$ (unit/ml) | lg $N_0$ | $T_7$ (unit/ml) | lg $N_7$ | $T_{14}$ (unit/ml) | lg $N_{14}$ | $T_{28}$ (unit/ml) | lg $N_{28}$ |
|---|---|---|---|---|---|---|---|---|
| S. aureus ATCC 6538 | $4,7 \times 10^5$ | 5,67 | <10 | 1 | <10 | 1 | <10 | 1 |
| E. coli ATCC 8739 | $4,2 \times 10^5$ | 5,62 | <10 | 1 | <10 | 1 | <10 | 1 |
| Ps. Aeruginosa ATCC 9027 | $4,0 \times 10^5$ | 5,60 | <10 | 1 | <10 | 1 | <10 | 1 |
| C. albicans ATCC 10231 | $3,6 \times 10^4$ | 4,55 | <10 | 1 | <10 | 1 | <10 | 1 |
| A. brasiliensis ATCC 16404 | $3,0 \times 10^4$ | 4,47 | <10 | 1 | <10 | 1 | <10 | 1 |

Table 4.

| The value of the reduction of the number of microorganisms in the sample at time intervals | | | |
|---|---|---|---|
| $R_x = lgN_0 - lgN_x$ | | | |
| Tested strains | $T_7$ | $T_{14}$ | T28 |
| S. aureus ATCC 6538 | 4,67 | 4,67 | 4,67 |
| E. coli ATCC 8739 | 4,62 | 4,62 | 4,62 |
| Ps. Aeruginosa ATCC 9027 | 4,60 | 4,60 | 4,60 |
| C. albicans ATCC 10231 | 3,55 | 3,55 | 3,55 |
| A. brasiliensis ATCC 16404 | 3,47 | 3,47 | 3,47 |
| Reduction values (Rx) are expressed in logarithmic units | | | |

Table 5. Assessment criteria for the maintenance effectiveness test according to the standard PN-EN ISO 11930:2019

| Microorganism | Bacteria | | | C. albicans | | | A. brasiliensis | |
|---|---|---|---|---|---|---|---|---|
| Time | $T_7$ | $T_{14}$ | $T_{28}$ | $T_7$ | $T_{14}$ | $T_{28}$ | $T_{14}$ | $T_{28}$ |
| Criterion A | ≥3 | ≥3 NI[a] | ≥3 NI | ≥1 | ≥1 NI | ≥1 NI | ≥0[b] | ≥1 |
| Criterion B | not tested | ≥3 | ≥3 NI | not tested | ≥1 | ≥1 NI | ≥0 | ≥0 NI |
| Reduction values calculated from the formula: $R_x = lgN_0 - lgN_x$ <br> [a] NI - the number of microorganisms does not increase <br> [b] $R_x = 0$ when $lgN_0 = lgN_x$ | | | | | | | | |

*Stability and Packaging Compatibility Testing*

The composition's stability and packaging compatibility were assessed. The results show that the prepared composition is a stable product (Table 6).

Table 6

| | Parameter | Requirements | Results |
|---|---|---|---|
| 1 | Character | Transparent, clear liquid, odorless | Compatible |
| 2 | pH | - | 5,5 |
| 3 | Mechanical contamination | does not contain | absent |
| 4 | Sample stability at -5°C 10 cycles: 1. Placing the sample at a reduced temperature (-5°C) for 24 hours 2. Taking the sample out for 8 hours - return to room temperature 3. Evaluation of any changes / mass destabilization / reactivity with packaging 4. Placing the sample at a reduced temperature for 16 hours until the next evaluation | No signs of mass destabilization, reactivity with packaging or other changes | No changes observed |
| 5 | Sample stability at room temperature +18°C/+20°C without light 10 cycles: 1. Assessment of possible changes / mass destabilization / reactivity with packaging every 24h | No signs of mass destabilization, reactivity with packaging or other changes | No changes observed |
| 6 | Stability at +38°C 10 cycles: 1. Placing the sample at an elevated temperature for 24 hours 2. Taking the sample out for 8 hours - return to room temperature 3. Evaluation of any changes / mass destabilization / reactivity with packaging 4. Placing the sample in specified temperature conditions for 16 hours until the next evaluation | No signs of mass destabilization, reactivity with packaging or other changes | No changes observed |
| 7 | Marking | Series number | Batch number (XX) and year of production (RR) in XXRR format Best before date - 24 months from the date of production |
| 8 | Stability of the preservation system: Sodium Benzoate + Potassium Sorbate | ISO 11930:2019 | Compatible |
| 9 | Compatibility of weight with packaging | complete | Compatible |

**Example 5**

Testing the Skin Tolerance and Soothing-Moisturizing Properties of the Inventive Composition

*Migration Test*

The migration test was performed using four-chamber inserts seeded with HaCaT cell line keratinocytes. The samples were then stimulated with the composition (IM2 and NE1 peptides, both at a concentration of 10 μg/ml, 1% poloxamer P407, preservative-free, already approved for use in cosmetics and food) and a peptide mixture. After 24 hours, the cells were fixed with paraformaldehyde and stained with crystal violet, and cell migration (cell-free surface area) was assessed using a light microscope. The experiment was repeated three times. A migration-stimulating effect was observed for the tested composition, as shown in Fig. 5.

*Dermatological studies of local skin tolerance*

The local skin tolerance of the composition prepared according to the invention was also assessed. The study aimed to assess the local skin tolerance of the composition in healthy volunteers and to determine any sensitizing or irritating properties. The selection of volunteers was conducted by a dermatologist after a physical and subjective examination of the volunteers, in accordance with the 1964 Declaration of Helsinki (as amended), Polish and EU regulations, and COLIPA guidelines, using inclusion and exclusion criteria. Twenty volunteers with dry, irritation-prone skin were selected for the study, including two individuals who had undergone radiotherapy and chemotherapy. The composition was applied to tissue discs, which were secured with a hypoallergenic patch on the back between the shoulder blades. The skin at the patch test site was normal, without any pathological changes. After reviewing the test procedure and receiving explanations, all subjects provided informed consent to participate in the study. The samples were removed after 48 hours. The first reading was taken after 24 hours, then immediately after sample removal, and then 72 hours after test application. Assessment was performed according to a generally accepted scale for dermatological testing. The subjects' skin and subjective sensations were assessed before, during, and after the test by a dermatologist. The composition was found to be free of irritant and sensitizing properties (Table 7).

Table 7. Results of application tests of the composition

| Probant | Age | Sex | Skin type | Results |
|---|---|---|---|---|
| 1 | 54 | M | M | (-) A |
| 2 | 49 | F | S | (-) A |
| 3 | 18 | F | T | (-) A |
| 4 | 23 | F | S | (-) A |
| 5 | 76 | M | M | (-) A, D |
| 6 | 76 | F | M | (-) A, D |
| 7 | 73 | F | N | (-) A, D |
| 8 | 70 | F | S | (-) A, D |
| 9 | 49 | F | S | (-) A, P |
| 10 | 72 | F | M | (-) A, P |
| 11 | 49 | F | S | (-) A |
| 12 | 49 | F | N | (-) A |
| 13 | 54 | M | N | (-) A |
| 14 | 50 | F | M | (-) A, R |
| 15 | 72 | F | S | (-) A, R |
| 16 | 18 | F | S | (-) A |
| 17 | 18 | F | S | (-) A |
| 18 | 18 | F | N | (-) A |
| 19 | 19 | F | S | (-) A |
| 20 | 23 | F | S | (-) A |

A - history of allergy, P - history of psoriasis, D - history of diabetes, R-radiotherapy

Sex: F- female, M - male

Skin type: N - normal, D - dry, M - mixed, T - oily

Patch test results assessed by a dermatologist:

(-) - negative result; no reaction

*Application studies to evaluate the effectiveness and functional benefits of the prepared composition*

Application studies were conducted on 20 volunteers. These were individuals aged 18-77 years with dry skin prone to atopy, allergies, and irritation, including two individuals who had undergone radiotherapy and chemotherapy, two with psoriasis, and four with a history of diabetes. The skin of the individuals enrolled in the study was normal at the time of study entry, with no lesions requiring pharmacological treatment. Participants received one package of the composition and used it for four weeks, as recommended, without using any other products for similar purposes during this time. Study results were obtained during application through direct interviews with users and after the study was completed from original questionnaires completed by the participants. The questions included in the survey were primarily closed-ended and concerned the functional properties of the composition and the condition of the skin after application.

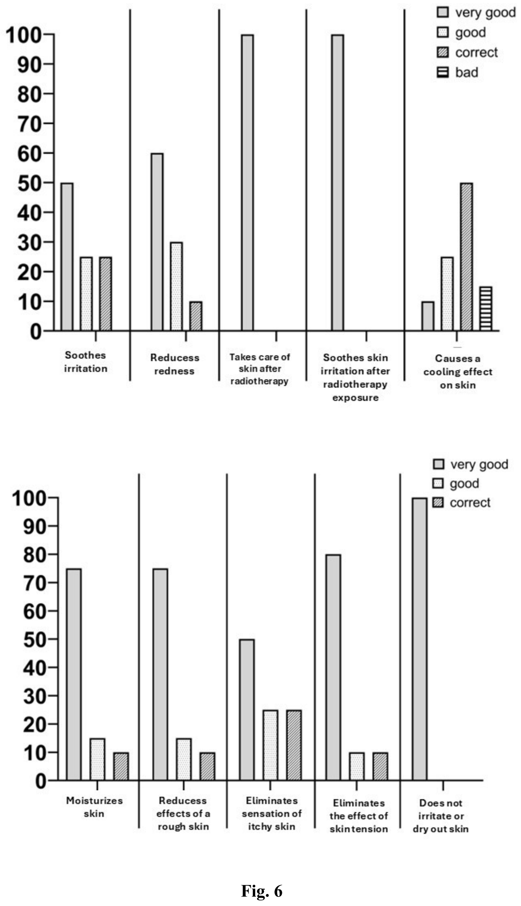

Based on the conducted studies, the composition was found to be very well tolerated. Additionally, it soothes irritation, reduces redness and rough skin, eliminates itching and tightness, and moisturizes and effectively nourishes sensitive skin. The tested composition also demonstrates practical benefits. The described results are presented in Fig. 6.

*Cytokine Secretion Assay*

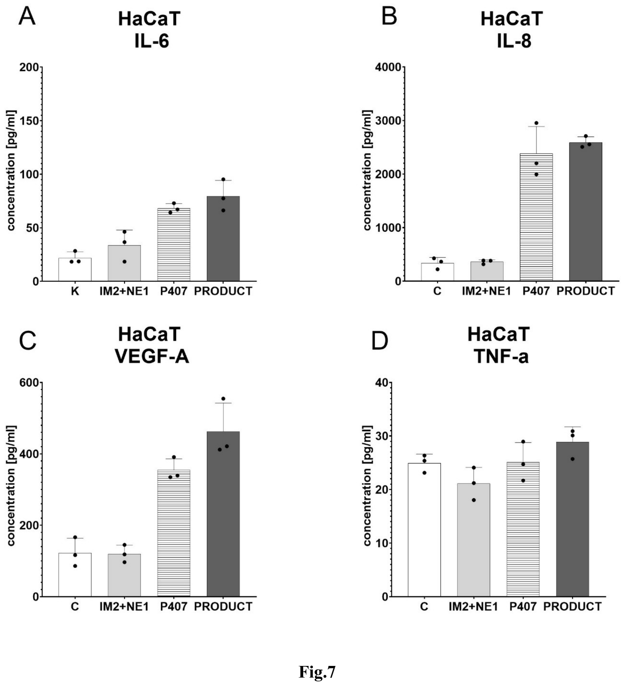

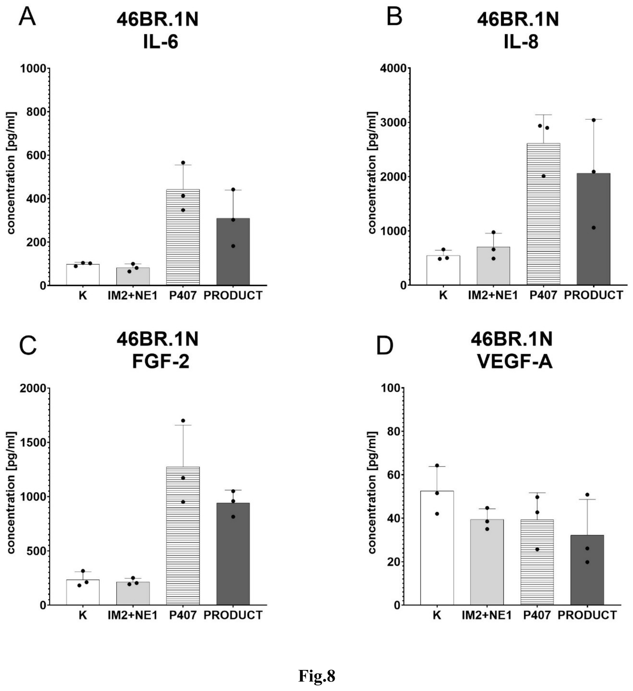

The secretory profile of the 46BR.1N and HaCaT cell lines was analyzed. Cells were seeded in 96-well plates. After 24 hours, the cells were stimulated with a mixture of the tested peptides (IM2 and NE1), poloxamer, and the composition (IM2 and NE1 peptides at 10 $\mu$g/ml, 1% poloxamer P407) at 100%. The following day, half of the medium was collected and frozen for further analysis. The experiment was performed in three independent replicates. On the day of the experiment, supernatants were thawed and Luminex analysis was performed according to the manufacturer's recommendations. The obtained results are presented in Figures 7-8.

An increase in the production of cytokines IL-6 and IL-8 was observed in HaCaT keratinocytes and 46BR.1N fibroblasts stimulated with poloxamer P407 and the composition. These interleukins are essential for the initial stage of wound healing. Increased levels of VEGF-$\alpha$ and TNF-$\alpha$ were also observed in the HaCaT cell line. Elevated levels of VEGF-$\alpha$ are crucial for angiogenesis and epidermal renewal in wounds. TNF-$\alpha$, on the other hand, is responsible for the formation of the extracellular matrix. Additionally, dermal fibroblasts had elevated levels of FGF-2 in their secretory profile. The presence of FGF-2 accelerates wound healing. The obtained secretory profile demonstrates cytokines with pro-regenerative activity in the HaCaT and 46BR.1N cell lines after application of the composition.

## Claims

1.   A pharmaceutical composition, **characterized in that** it comprises IM2 peptide of SEQ ID NO: 1 and NE1 peptide of SEQ ID NO: 2, and at least one pharmaceutically acceptable excipient.

2.   The pharmaceutical composition according to claim 1, wherein the concentration of the NE1 peptide is from 0.1 to 25 $\mu$g/ml and the concentration of the IM2 is from 0.1 to 150 $\mu$g/ml.

3.   The pharmaceutical composition according to claim 1, wherein the poloxamer P-407 concentration is 1% by weight based on the total weight of the composition.

4.   The pharmaceutical composition according to any one of claims 1 to 3, wherein the preservative is from 1% to 1.5% by weight based on the total weight of the composition.

5.   The pharmaceutical composition according to claim 4, wherein the preservative is a mixture of potassium sorbate and sodium benzoate.

6.   The pharmaceutical composition according to any claim 1-5, wherein the composition is in the form of an emulsion, aerosol, or aqueous solution.

7.   The pharmaceutical composition according to any claim 1-6, wherein the composition is in the form of an external preparation for skin care and protection following radiation therapy, burns, and other irritants such as sunlight or temperature.

8. The composition according to claim 7, wherein the composition is in the form of an aerosol.

**A**

MS Spectrum Graph

#:1   Ret.Time:Averaged 4.590-5.095(Scan#:919-1020)
BG Mode:None
Mass Peaks:9   Base Peak:332.65(12269428)   MS Stage:MS   Polarity:Pos   Segment1 - Event1   Precursor:-----   Cutoff:   Ionization Mode:ESI

**B**

MS Spectrum Graph

#:1   Ret.Time:Averaged 6.130-6.140(Scan#:1227-1229)
BG Mode:Calc 6.045<->6.290(1210<->1259)
Mass Peaks:3   Base Peak:372.65(4985220)   MS Stage:MS   Polarity:Pos   Segment1 - Event1   Precursor:-----   Cutoff:   Ionization Mode:ESI

**Fig. 1**

**XTT HaCaT**

**XTT 46BR.1N**

**XTT primary fibroblasts**

**Fig.2**

**Fig.3**

**Fig. 4**

**Fig.5**

**Fig. 6**

**Fig.7**

Fig.8

Europäisches
Patentamt
European
Patent Office
Office européen
des brevets

# EUROPEAN SEARCH REPORT

**Application Number**

EP 25 20 6946

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SAWICKA JUSTYNA ET AL: "Functionalized Peptide Fibrils as a Scaffold for Active Substances in Wound Healing", INTERNATIONAL JOURNAL OF MOLECULAR SCIENCES, vol. 22, no. 8, 7 April 2021 (2021-04-07), page 3818, XP093278707, Basel, CH ISSN: 1422-0067, DOI: 10.3390/ijms22083818 * p. 3, par. 2; Table 1 * ----- | 1-8 | INV. A61K38/00 A61K8/64 A61P17/02 A61P17/16 C07K14/00 |
| Y | PL 237 001 B1 (UNIV GDANSKI [PL]; POLITECHNIKA GDANSKA [PL] ET AL.) 8 March 2021 (2021-03-08) * p. 5, par. 5; claim 3 * ----- | 1-8 | |
| Y | HAIDARI HANIF ET AL: "Therapeutic potential of antimicrobial peptides for treatment of wound infection", AMERICAN JOURNAL OF PHYSIOLOGY-CELL PHYSIOLOGY, vol. 324, no. 1, 21 November 2022 (2022-11-21), pages C29-C38, XP093368954, ISSN: 0363-6143, DOI: 10.1152/ajpcell.00080.2022 * C30, col. 2, par. 2 * ----- -/-- | 1-8 | TECHNICAL FIELDS SEARCHED (IPC) A61K C07K A61P |

The present search report has been drawn up for all claims

| Place of search | Date of completion of the search | Examiner |
|---|---|---|
| Munich | 24 February 2026 | Seregélyes, Csaba |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 25 20 6946

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DEPTULA M. ET AL: "Development and evaluation of RADA-PDGF2 self-assembling peptide hydrogel for enhanced skin wound healing", FRONTIERS IN PHARMACOLOGY, vol. 14, 28 November 2023 (2023-11-28), XP093368593, CH ISSN: 1663-9812, DOI: 10.3389/fphar.2023.1293647 * p. 2, col. 2, par. 2; p. 12, col. 2, par. 1 * | 1-8 | |
| Y | LOREN PICKART ET AL: "Regenerative and Protective Actions of the GHK-Cu Peptide in the Light of the New Gene Data", INTERNATIONAL JOURNAL OF MOLECULAR SCIENCES, vol. 19, no. 7, 7 July 2018 (2018-07-07), page 1987, XP055713431, DOI: 10.3390/ijms19071987 * p. 1, par. 1 * | 1-8 | |
| Y | SAWICKA JUSTYNA ET AL: "Imunofan-RDKVYR Peptide-Stimulates Skin Cell Proliferation and Promotes Tissue Repair", MOLECULES, vol. 25, no. 12, 23 June 2020 (2020-06-23), page 2884, XP093368238, CH ISSN: 1420-3049, DOI: 10.3390/molecules25122884 * Abstract * | 1-8 | TECHNICAL FIELDS SEARCHED (IPC) |
| A | US 2018/193406 A1 (CHO DAE HO [KR] ET AL) 12 July 2018 (2018-07-12) * par. 81 * | 1-8 | |

The present search report has been drawn up for all claims

| Place of search | Date of completion of the search | Examiner |
|---|---|---|
| Munich | 24 February 2026 | Seregélyes, Csaba |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................

& : member of the same patent family, corresponding document

EPO FORM 1503 03.82 (P04C01)

**page 2 of 2**

## ANNEX TO THE EUROPEAN SEARCH REPORT
## ON EUROPEAN PATENT APPLICATION NO.

EP 25 20 6946

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2026

| Patent document cited in search report | | Publication date | Patent family member(s) | | Publication date |
|---|---|---|---|---|---|
| PL 237001 | B1 | 08-03-2021 | NONE | | |
| US 2018193406 | A1 | 12-07-2018 | CN | 109563132 A | 02-04-2019 |
| | | | EP | 3444265 A2 | 20-02-2019 |
| | | | ES | 2835124 T3 | 21-06-2021 |
| | | | JP | 6573295 B2 | 11-09-2019 |
| | | | JP | 2018528978 A | 04-10-2018 |
| | | | KR | 20170113156 A | 12-10-2017 |
| | | | US | 2018193406 A1 | 12-07-2018 |
| | | | WO | 2017164609 A2 | 28-09-2017 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- PL 237242 B1
- PL 237001 B1

- PL 241125 B1

**Non-patent literature cited in the description**

- **L. LORENZO-GALLEGO et al.** Changes in Pain Sensitivity in Treatment for Breast Cancer: A 12-Month Follow-Up Case Series. *Int. J. Environ. Res. Public Health*, 2022, vol. 19 (7), 4055
- **S. A. BASKIN et al.** The neoadjuvant approach to treatment of breast cancer: Multidisciplinary management to improve outcomes. *Surgical Oncology Insight*, 2024, vol. 1, 100059

- **M.D. YUE-YUNG HU et al.** Impact of neoadjuvant chemotherapy on breast reconstruction. *Cancer*, 2011, vol. 117 (13), 2827-3060
- **H. MATSUKI et al.** Pitfall in the Surgical Management of a Shrunken Skin Defect after Neoadjuvant Chemotherapy for Locally Advanced Breast Cancer. *Case Rep Oncol*, 2022, vol. 15 (3), 1101-1106